# EUROPEAN PATENT SPECIFICATION

(11) **EP 3 990 064 B1**
(45) Date of publication and mention of the grant of the patent: **09.10.2024**
(21) Application number: 20735104.0
(22) Date of filing: 23.06.2020
(51) Int. Cl.: B65C 3/16, A61M 5/50, G09F 3/00, A61M 39/10

(54) **INJECTION DEVICE**
INJEKTIONSVORRICHTUNG
DISPOSITIF D'INJECTION

(30) Priority: 26.06.2019 EP 19305851
(43) Date of publication of application: 04.05.2022
(73) Proprietor: Sanofi, 75017 Paris (FR)
(72) Inventor: JUGL, Michael, 65926 Frankfurt am Main (DE); POMMEREAU, Christian, 65926 Frankfurt am Main (DE); SCHMITT, Jochen, 65926 Frankfurt am Main (DE)
(74) Representative: Keil & Schaafhausen Patentanwälte PartGmbB
(86) International application number: PCT/EP2020/067392
(87) International publication number: WO 2020/260220

(56) References cited:
- EP-A1- 1 882 486
- WO-A2-2012/017063
- US-A- 4 565 543
- US-A- 5 263 933
- US-A1- 2017 007 766

## Description

The present invention is generally directed to a drug delivery device, e.g. an injection device for selecting and dispensing a number of user variable doses of a medicament, especially to a disposable injection device.

Pen type drug delivery devices have application where regular injection by persons without formal medical training occurs. This may be increasingly common among patients having diabetes where self-treatment enables such patients to conduct effective management of their disease. In practice, such a drug delivery device allows a user to individually select and dispense a number of user variable doses of a medicament. The present invention is further directed to auto-injectors, syringes and so called fixed dose devices which only allow dispensing of a predefined dose without the possibility to increase or decrease the set dose.

There are basically two types of drug delivery devices: resettable devices (i.e., reusable) and non-resettable (i.e., disposable). For example, disposable pen delivery devices are supplied as self-contained devices. Such self-contained devices do not have removable pre-filled cartridges. Rather, the pre-filled cartridges may not be removed and replaced from these devices without destroying the device itself. Consequently, such disposable devices need not have a resettable dose setting mechanism. The present invention is directed to disposable devices.

These types of pen delivery devices (so named because they often resemble an enlarged fountain pen) generally comprise three primary elements: a cartridge section that includes a cartridge often contained within a housing or holder; a needle assembly connected to one end of the cartridge section; and a dosing section connected to the other end of the cartridge section. A cartridge (often referred to as an ampoule) typically includes a reservoir that is filled with a medication (e.g., insulin), a movable rubber type bung or stopper located at one end of the cartridge reservoir, and a top having a pierceable rubber seal located at the other, often necked-down, end. A crimped annular metal band is typically used to hold the rubber seal in place. While the cartridge housing may be typically made of plastic, cartridge reservoirs have historically been made of glass.

The needle assembly is typically a replaceable double-ended needle assembly. Before an injection, a replaceable double-ended needle assembly is attached to one end of the cartridge assembly, a dose is set, and then the set dose is administered. Such removable needle assemblies may be threaded onto, or pushed (i.e., snapped) onto the pierceable seal end of the cartridge assembly.

The dosing section or dose setting mechanism is typically the portion of the pen device that is used to set (select) a dose. During an injection, a spindle or piston rod contained within the dose setting mechanism presses against the bung or stopper of the cartridge. This force causes the medication contained within the cartridge to be injected through an attached needle assembly. After an injection, as generally recommended by most drug delivery device and/or needle assembly manufacturers and suppliers, the needle assembly is removed and discarded.

A further differentiation of drug delivery device types refers to the drive mechanism: There are devices which are manually driven, e.g. by a user applying a force to an injection button, devices which are driven by a spring or the like and devices which combine these two concepts, i.e. spring assisted devices which still require a user to exert an injection force. The spring-type devices involve springs which are preloaded and springs which are loaded by the user during dose selecting. Some stored-energy devices use a combination of spring preload and additional energy provided by the user, for example during dose setting.

WO 2012/020084 A2 discloses a reusable drug delivery device with a cartridge holder containing a cartridge or the like reservoir filled with a medicament. If the medicament in the cartridge is expended, the cartridge may be replaced. To exchange an empty cartridge, the user is called upon to remove the cartridge holder from the dose setting mechanism. A heat shrink label is positioned over a portion of the cartridge and a portion of a connector such that the heat shrink member maintains the connector on the reservoir. The connector is dimensioned so as to cooperate with the drug delivery device, either the cartridge holder or the dose setting member of the drug delivery device to thereby prevent inadvertent cross use of cartridge assemblies and therefore inadvertent cross use of medicaments.

An example of a disposable injection device having an outer housing which contains a dose setting mechanism is known from WO 2014/033197 A1. In this device, the outer housing is a generally tubular element having a distal part which forms a cartridge holder for receiving a cartridge and a proximal part which forms an outer body. The outer housing is transparent and the outer body is provided with an opaque layer. The opaque layer covers most of the outer body with the exception of a transparent window.

In disposable drug delivery devices the dose setting mechanism is typically adapted to the cartridge and the medicament contained therein such that the user is able to precisely set and dispense a certain amount of the medicament with the drug delivery device. Thus, changing the cartridge may have a negative effect on dose accuracy and, hence, may result in health problems. There is a need for preventing users from replacing an empty cartridge in a disposable drug delivery device by a cartridge not suitable for the respective dose setting mechanism. There is a further need to display any attempts of manipulating drug delivery devices.

US 2017/007766 A1, EP 1 882 486 A1 and US 4,565,543 A each disclose a drug delivery device. From these documents the use of an, e.g. heat shrinkable, label is known which may be shrunk onto portions of a syringe or a medicament cartridge.

It is therefore an object of the present invention to provide a an improved injection device which a mechanism that prevents the unintentional use of a manipulated device.

This object is solved by an injection device as defined in claim 1.

An injection device preferably comprises a housing containing a dose setting mechanism, a cartridge holder containing a cartridge with a medicament and a heat shrinkable label which comprises information and/or symbols about the medicament. In a disposable injection device according to one aspect of the present invention the cartridge holder is inseparably locked to the housing. In other words, in such a disposable injection device the housing and the cartridge holder are unitarily formed, locked or permanently attached to each other such that the cartridge holder is not supposed to be detached by a user of the injection device from the housing. If the heat shrinkable label is heat shrunk on the housing and the cartridge holder such that it at least partially covers the housing and the cartridge holder, the label may be used to show whether the device is in its original condition. A broken label indicates that the device may have been manipulated. According to an embodiment a label at least partially covering the housing and the cartridge holder may be a label which at least covers an interface between the cartridge holder and the housing or a bridging or transition zone between the cartridge holder and the housing.

In a disposable injection device the housing and the cartridge holder may be formed as a unitary component part. As an alternative, the cartridge holder may be permanently attached to the housing, for example by means of snap features, welding, gluing or the like. In addition to the indication whether the device may have been manipulated, the provision of a heat shrinkable label provides additional strength preventing detachment of the cartridge holder from the housing.

In conventional injection devices information and/or symbols about the medicament are often provided by means of an adhesive label or by printing on the outer surface of the device. Placing an adhesive label at the correct position on device is sometimes difficult and depending on the materials used for the housing or the cartridge holder it may be difficult to permanently provide information and/or symbols by printing. In contrast to that a heat shrinkable label has the additional benefit of facilitating the provision of information and/or symbols permanently. The information and/or symbols may not only include information about the medicament, like the name of the medicament and/or the manufacturer and/or distributor, the name, concentration and/or amount of active ingredients, production and/or expiry dates, but further may include at least one color code, at least one scale, at least one frame, at least one pointer, or the like. The information and/or symbols may be visible and/or tactile, e.g. in Braille. Further, the information and/or symbols may be provided permitting haptic differentiation.

According to an aspect of the present invention, the heat shrinkable label has the form of a jacket at least partially encasing the housing and the cartridge holder. With such a sleeve-like configuration of the label it is easy to insert the device into the jacket, thereby placing the label in a predefined position. The sleeve-like form of a jacket is suitable for fully encasing the area of the device in which the cartridge holder is attached to the housing or in which the cartridge holder continues into the housing. Thus, a label in the form of a jacket is suitable for shielding an area of the device where any manipulation is most likely to occur.

The heat shrinkable label may comprises at least a transparent or translucent portion, which is positioned on the housing overlapping a window provided in the housing. In addition or as an alternative, the heat shrinkable label may form a window covering an aperture in the housing. Such a window is typically used in combination with the dose setting mechanism for displaying the currently set dose of the device. For example, a number sleeve or a display sleeve may move with respect to the housing during dose setting of the device such that a number or symbol corresponding to the set dose is visible through the window or a poacher of the housing.

The information and/or symbols are preferably printed on the inner surface of the heat shrinkable label. In contrast to conventional adhesive labels this prevents the information on the label from being lost due to wear and tear. For example, the information and/or symbols comprises a scale positioned on the cartridge holder and information about the medicament positioned on the housing. This facilitates production of the cartridge holder and the housing because separate provision of information and/or symbols on the cartridge holder and the housing and alignment is not required. Further, the outer surface of the heat shrinkable label may be fully or partially lacquered to give the device.

Preferably, the heat shrinkable label consists of PET (Polyethylene terephthalate) or OPP (oriented polypropylene). In addition or as an alternative, the heat shrinkable label consists of and/or is provided with a layer inhibiting the transmission of actinic light. This has the benefit of protecting the medicament contained in the cartridge. Further, the heat shrinkable label may have a photochromic functionality, e.g. providing a color change due to exposure to solar light. Still further, the heat shrinkable label may consist of a biodegradable material.

Further, the heat shrinkable label may comprise an electronic component and/or maintains an electronic component on the housing and/or on the cartridge holder. For example, the heat shrinkable label may comprise a temperature measuring strip. In a further example, the heat shrinkable label may position and maintain an add-on device or component part in or on the disposable injection device, preferably on the housing thereof. Such an add-on device or component part may include a communication unit for sending and/or receiving data, a display unit for indicating a target dose value and/or an actual dose value, a read-out unit for recording values, at least one sensor or for detecting the position, state, movement, direction of movement and/or acceleration of at least one component part of the injection device, or the like. The heat shrinkable label may be used as the only fastener for such a component or may be used in addition to further attaching means.

According to a further aspect of the invention the heat shrinkable label may comprise at least one weakened line permitting severance and removal of a portion of the heat shrinkable label. A weakened line may be a perforated line, an incised line, an interrupted line and/or a line of less thickness or made from a weaker material. The at least one weakened line may be provided with a tear-off strip or tap facilitating severance of the heat shrinkable label along a predefined line. The at least one weakened line may extend e.g. axially, circumferentially and/or radially.

For example, the cartridge holder comprises an interface for attaching a needle on the cartridge holder, wherein the heat shrinkable label comprises a first cap portion covering the interface for attaching a needle and wherein the at least one weakened line is positioned permitting severance and removal of the first cap portion. In addition or as an alternative, the dose setting mechanism may comprise a dose setting member protruding from the housing, wherein the heat shrinkable label comprises a second cap portion covering the dose setting member and wherein the at least one weakened line is positioned permitting severance and removal of the second cap portion. Still further, the disposable injection device may comprise a component part which has to be activated and/or manipulated for starting the dose setting procedure and/or the dose dispensing procedure. This component part may be a separate component part or may be identical with the dose setting member. In the case where this component part is a separate part a portion of the heat shrinkable label which is provided with a weakened line may cover this component part. In other words, a portion of the heat shrinkable label may be used as a tamper-evident closure for the device or a portion thereof.

According to the present invention, the label is heat shrunk onto the injection device. In addition, the heat shrinkable label may be glued or welded onto the housing and/or onto the cartridge holder.

In an embodiment of the present invention, the dose setting mechanism of the injection device is generally designed and functions as described in WO 2014/033197 A1.

The injection device typically comprises a cartridge containing a medicament. The term "medicament", as used herein, means a pharmaceutical formulation containing at least one pharmaceutically active compound,
wherein in one embodiment the pharmaceutically active compound has a molecular weight up to 1500 Da and/or is a peptide, a proteine, a polysaccharide, a vaccine, a DNA, a RNA, an enzyme, an antibody or a fragment thereof, a hormone or an oligonucleotide, or a mixture of the above-mentioned pharmaceutically active compound,
wherein in a further embodiment the pharmaceutically active compound is useful for the treatment and/or prophylaxis of diabetes mellitus or complications associated with diabetes mellitus such as diabetic retinopathy, thromboembolism disorders such as deep vein or pulmonary thromboembolism, acute coronary syndrome (ACS), angina, myocardial infarction, cancer, macular degeneration, inflammation, hay fever, atherosclerosis and/or rheumatoid arthritis,
wherein in a further embodiment the pharmaceutically active compound comprises at least one peptide for the treatment and/or prophylaxis of diabetes mellitus or complications associated with diabetes mellitus such as diabetic retinopathy,
wherein in a further embodiment the pharmaceutically active compound comprises at least one human insulin or a human insulin analogue or derivative, glucagon-like peptide (GLP-1) or an analogue or derivative thereof, or exendin-3 or exendin-4 or an analogue or derivative of exendin-3 or exendin-4.

Insulin analogues are for example Gly(A21), Arg(B31), Arg(B32) human insulin; Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Insulin derivates are for example B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-Y-glutamyl)-des(B30) human insulin; B29-N-(N-lithocholyl-Y-glutamyl)-des(B30) human insulin; B29-N-(w-carboxyheptadecanoyl)-des(B30) human insulin and B29-N-(w-carboxyheptadecanoyl) human insulin.

Exendin-4 for example means Exendin-4(1-39), a peptide of the sequence H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp- Leu- Lys-Asn-Gly-G ly- Pro-Ser-Ser-G ly-Ala- Pro- Pro- Pro-Ser- N H2.

Exendin-4 derivatives are for example selected from the following list of compounds:
H-(Lys)4-des Pro36, des Pro37 Exendin-4(1-39)-NH2,
H-(Lys)5-des Pro36, des Pro37 Exendin-4(1-39)-NH2,
des Pro36 Exendin-4(1-39),
des Pro36 [Asp28] Exendin-4(1-39),
des Pro36 [lsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(O2)25, Asp28] Exendin-4(1-39),
des Pro36 [Trp(O2)25, IsoAsp28] Exendin-4(1-39),
des Pro36 [Met(O)14 Trp(O2)25, Asp28] Exendin-4(1-39),
des Pro36 [Met(O)14 Trp(O2)25, IsoAsp28] Exendin-4(1-39); or
des Pro36 [Asp28] Exendin-4(1-39),
des Pro36 [lsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(O2)25, Asp28] Exendin-4(1-39),
des Pro36 [Trp(O2)25, IsoAsp28] Exendin-4(1-39),
des Pro36 [Met(O)14 Trp(O2)25, Asp28] Exendin-4(1-39),
des Pro36 [Met(O)14 Trp(O2)25, IsoAsp28] Exendin-4(1-39),
wherein the group -Lys6-NH2 may be bound to the C-terminus of the Exendin-4 derivative;
or an Exendin-4 derivative of the sequence
des Pro36 Exendin-4(1-39)-Lys6-NH2 (AVE0010),
H-(Lys)6-des Pro36 [Asp28] Exendin-4(1-39)-Lys6-NH2,
des Asp28 Pro36, Pro37, Pro38Exendin-4(1-39)-NH2,
H-(Lys)6-des Pro36, Pro38 [Asp28] Exendin-4(1-39)-NH2,
H-Asn-(Glu)5des Pro36, Pro37, Pro38 [Asp28] Exendin-4(1-39)-NH2,
des Pro36, Pro37, Pro38 [Asp28] Exendin-4(1-39)-(Lys)6-NH2,
H-(Lys)6-des Pro36, Pro37, Pro38 [Asp28] Exendin-4(1-39)-(Lys)6-NH2,
H-Asn-(Glu)5-des Pro36, Pro37, Pro38 [Asp28] Exendin-4(1-39)-(Lys)6-NH2,
H-(Lys)6-des Pro36 [Trp(O2)25, Asp28] Exendin-4(1-39)-Lys6-NH2,
H-des Asp28 Pro36, Pro37, Pro38 [Trp(O2)25] Exendin-4(1-39)-NH2,
H-(Lys)6-des Pro36, Pro37, Pro38 [Trp(O2)25, Asp28] Exendin-4(1-39)-NH2,
H-Asn-(Glu)5-des Pro36, Pro37, Pro38 [Trp(O2)25, Asp28] Exendin-4(1-39)-NH2,
des Pro36, Pro37, Pro38 [Trp(O2)25, Asp28] Exendin-4(1-39)-(Lys)6-NH2,
H-(Lys)6-des Pro36, Pro37, Pro38 [Trp(O2)25, Asp28] Exendin-4(1-39)-(Lys)6-NH2,
H-Asn-(Glu)5-des Pro36, Pro37, Pro38 [Trp(O2)25, Asp28] Exendin-4(1-39)-(Lys)6-NH2,
H-(Lys)6-des Pro36 [Met(O)14, Asp28] Exendin-4(1-39)-Lys6-NH2,
des Met(O)14 Asp28 Pro36, Pro37, Pro38 Exendin-4(1-39)-NH2,
H-(Lys)6-desPro36, Pro37, Pro38 [Met(O)14, Asp28] Exendin-4(1-39)-NH2,
H-Asn-(Glu)5-des Pro36, Pro37, Pro38 [Met(O)14, Asp28] Exendin-4(1-39)-NH2,
des Pro36, Pro37, Pro38 [Met(O)14, Asp28] Exendin-4(1-39)-(Lys)6-NH2,
H-(Lys)6-des Pro36, Pro37, Pro38 [Met(O)14, Asp28] Exendin-4(1-39)-(Lys)6-NH2,
H-Asn-(Glu)5 des Pro36, Pro37, Pro38 [Met(O)14, Asp28] Exendin-4(1-39)-(Lys)6-NH2,
H-Lys6-des Pro36 [Met(O)14, Trp(O2)25, Asp28] Exendin-4(1-39)-Lys6-NH2,
H-des Asp28 Pro36, Pro37, Pro38 [Met(O)14, Trp(O2)25] Exendin-4(1-39)-NH2,
H-(Lys)6-des Pro36, Pro37, Pro38 [Met(O)14, Asp28] Exendin-4(1-39)-NH2,
H-Asn-(Glu)5-des Pro36, Pro37, Pro38 [Met(O)14, Trp(O2)25, Asp28] Exendin-4(1-39)-NH2, des Pro36, Pro37, Pro38 [Met(O)14, Trp(O2)25, Asp28] Exendin-4(1-39)-(Lys)6-NH2,
H-(Lys)6-des Pro36, Pro37, Pro38 [Met(O)14, Trp(O2)25, Asp28] Exendin-4(S1-39)-(Lys)6-NH2,
H-Asn-(Glu)5-des Pro36, Pro37, Pro38 [Met(O)14, Trp(O2)25, Asp28] Exendin-4(1-39)-(Lys)6-NH2;
or a pharmaceutically acceptable salt or solvate of any one of the afore-mentioned Exendin-4 derivative.

Hormones are for example hypophysis hormones or hypothalamus hormones or regulatory active peptides and their antagonists as listed in Rote Liste, ed. 2008, Chapter 50, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, Goserelin.

A polysaccharide is for example a glucosaminoglycane, a hyaluronic acid, a heparin, a low molecular weight heparin or an ultra low molecular weight heparin or a derivative thereof, or a sulphated, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium.

Antibodies are globular plasma proteins (-150 kDa) that are also known as immunoglobulins which share a basic structure. As they have sugar chains added to amino acid residues, they are glycoproteins. The basic functional unit of each antibody is an immunoglobulin (Ig) monomer (containing only one Ig unit); secreted antibodies can also be dimeric with two Ig units as with IgA, tetrameric with four Ig units like teleost fish IgM, or pentameric with five Ig units, like mammalian IgM.

The Ig monomer is a "Y"-shaped molecule that consists of four polypeptide chains; two identical heavy chains and two identical light chains connected by disulfide bonds between cysteine residues. Each heavy chain is about 440 amino acids long; each light chain is about 220 amino acids long. Heavy and light chains each contain intrachain disulfide bonds which stabilize their folding. Each chain is composed of structural domains called Ig domains. These domains contain about 70-110 amino acids and are classified into different categories (for example, variable or V, and constant or C) according to their size and function. They have a characteristic immunoglobulin fold in which two β sheets create a "sandwich" shape, held together by interactions between conserved cysteines and other charged amino acids.

There are five types of mammalian Ig heavy chain denoted by α, δ, ε, γ, and µ. The type of heavy chain present defines the isotype of antibody; these chains are found in IgA, IgD, IgE, IgG, and IgM antibodies, respectively.

Distinct heavy chains differ in size and composition; α and γ contain approximately 450 amino acids and δ approximately 500 amino acids, while µ and ε have approximately 550 amino acids. Each heavy chain has two regions, the constant region (CH) and the variable region (VH). In one species, the constant region is essentially identical in all antibodies of the same isotype, but differs in antibodies of different isotypes. Heavy chains γ, α and δ have a constant region composed of three tandem Ig domains, and a hinge region for added flexibility; heavy chains µ and ε have a constant region composed of four immunoglobulin domains. The variable region of the heavy chain differs in antibodies produced by different B cells, but is the same for all antibodies produced by a single B cell or B cell clone. The variable region of each heavy chain is approximately 110 amino acids long and is composed of a single Ig domain.

In mammals, there are two types of immunoglobulin light chain denoted by λ and κ. A light chain has two successive domains: one constant domain (CL) and one variable domain (VL). The approximate length of a light chain is 211 to 217 amino acids. Each antibody contains two light chains that are always identical; only one type of light chain, κ or λ, is present per antibody in mammals.

Although the general structure of all antibodies is very similar, the unique property of a given antibody is determined by the variable (V) regions, as detailed above. More specifically, variable loops, three each the light (VL) and three on the heavy (VH) chain, are responsible for binding to the antigen, i.e. for its antigen specificity. These loops are referred to as the Complementarity Determining Regions (CDRs). Because CDRs from both VH and VL domains contribute to the antigen-binding site, it is the combination of the heavy and the light chains, and not either alone, that determines the final antigen specificity.

An "antibody fragment" contains at least one antigen binding fragment as defined above, and exhibits essentially the same function and specificity as the complete antibody of which the fragment is derived from. Limited proteolytic digestion with papain cleaves the Ig prototype into three fragments. Two identical amino terminal fragments, each containing one entire L chain and about half an H chain, are the antigen binding fragments (Fab). The third fragment, similar in size but containing the carboxyl terminal half of both heavy chains with their interchain disulfide bond, is the crystalizable fragment (Fc). The Fc contains carbohydrates, complement-binding, and FcR-binding sites. Limited pepsin digestion yields a single F(ab')2 fragment containing both Fab pieces and the hinge region, including the H-H interchain disulfide bond. F(ab')2 is divalent for antigen binding. The disulfide bond of F(ab')2 may be cleaved in order to obtain Fab'. Moreover, the variable regions of the heavy and light chains can be fused together to form a single chain variable fragment (scFv).

Pharmaceutically acceptable salts are for example acid addition salts and basic salts. Acid addition salts are e.g. HCl or HBr salts. Basic salts are e.g. salts having a cation selected from alkali or alkaline, e.g. Na+, or K+, or Ca2+, or an ammonium ion N+(R1)(R2)(R3)(R4), wherein R1 to R4 independently of each other mean: hydrogen, an optionally substituted C1-C6-alkyl group, an optionally substituted C2-C6-alkenyl group, an optionally substituted C6-C10-aryl group, or an optionally substituted C6-C10-heteroaryl group. Further examples of pharmaceutically acceptable salts are described in "Remington's Pharmaceutical Sciences" 17. ed. Alfonso R. Gennaro (Ed.), Mark Publishing Company, Easton, Pa., U.S.A., 1985 and in Encyclopedia of Pharmaceutical Technology.

Pharmaceutically acceptable solvates are for example hydrates.

Non-limiting, exemplary embodiments of the invention will now be described with reference to the accompanying drawings, in which:
- Figure 1: schematically shows an injection device according to the prior art;
- Figure 2: schematically shows the injection device according to the prior art with the cap removed and a dose set; and
- Figure 3: schematically shows a label to be a heat shrunk onto the injection device according to Figure 1.

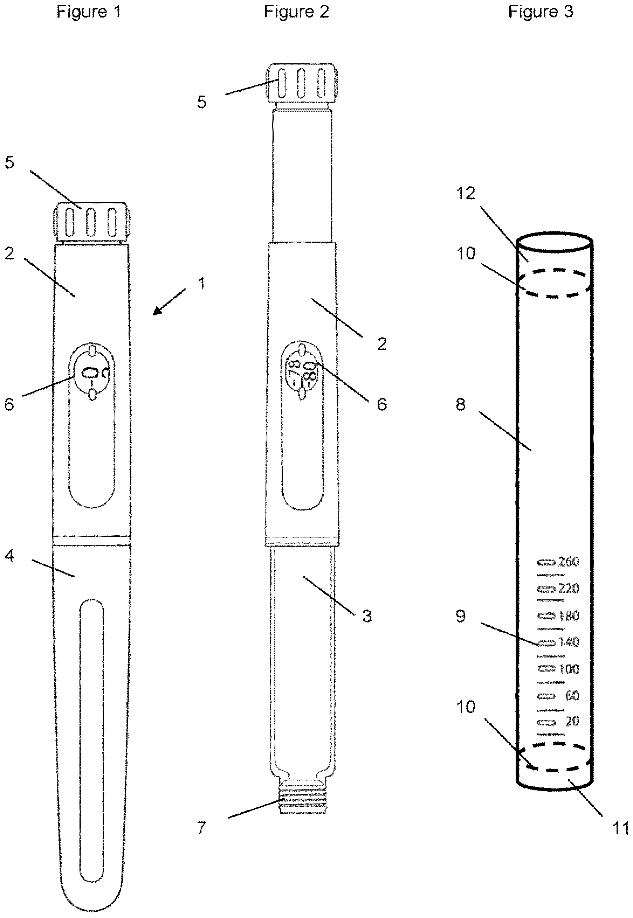

Referring to Figures 1 and 2, a disposable injection device as disclosed in WO 2014/033197 A1 is shown. The device has a distal end (upper end in Figure 1) and a proximal end (lower end in Figure 1). The injection device 1 comprises a housing 2 and a cartridge holder 3 which is a firmly connected to the housing 2 or which is an integral part of the housing 2. A cartridge (not shown) containing a medicament is received in the cartridge holder 3. In Figure 1 a detachable cap 4 covers the cartridge holder 3. The cap 4 is removed in Figure 2.

The disposable injection device 1 further comprises a dose setting mechanism provided in the housing 2. A dose setting member 5 of the dose setting mechanism protrudes from the housing 2 such that a user may grip and move the dose setting member 5 relative to the housing 2 during dose setting. Figure 1 depicts a state with a dose of zero set which dose is visible through the window or aperture 6 in the housing 2, i.e. an at rest state of the device. In contrast to that, in Figure 2 a larger dose has been set by moving, e.g. rotating, the dose setting member 5 with respect to the housing 2.

The distal end of the cartridge holder 3 is provided with a threaded interface 7 for attaching a needle arrangement (not shown) to the cartridge holder.

Figure 3 depicts a transparent heat shrinkable label 8 in the form of a transparent jacket into which the housing 2 and the cartridge holder 3 may be inserted. The heat shrinkable label 8 can be firmly attached to the injection device 1 by heat shrinking the label 8 onto the housing 2 and the cartridge holder 3. When applying heat to the label 8, the label shrinks and adapts to the outer contour of the housing 2 and the cartridge holder 3. Thus, the label 8 firmly fits over the housing 2 and the cartridge holder 3. In addition, the label 8 may be further fixed on the housing 2 and the cartridge holder 3 by an adhesive. With the label 8 at least partially covering the housing 2 and the cartridge holder 3, it would be required to remove the label 8 prior to forcibly detaching the cartridge holder 3 from the housing 2 or destroying same.

The heat shrinkable label 8 is made from a material which is sufficiently strong and durable to maintain the label 8 on the disposable injection device 1 for the intended use of the device. Suitable materials include but are not limited to PET or OPP. The heat shrinkable label 8 may consist of a material inhibiting the transmission of actinic light and/or may be provided with a layer inhibiting the transmission of actinic light. Preferably, the heat shrinkable label 8 consists of a single layer of material which may be printed and/or lacquered. As an alternative, the heat shrinkable label 8 may comprise several layers of material.

In the exemplary embodiment depicted in Figure 3 the scale 9 is printed on the inner side of the label 8 such that the scale 9 is visible through the transparent label 8. Further, the transparent label 8 allows a user to read the dose numbers visible through the window or aperture 6 of the housing 2. In addition or as an alternative, a further information and/or symbols, e.g. about the medicament, may be printed on the inner side of the label 8. Still further, the outer side of the label 8 may be at least partially lacquered. A weakened line 10 in the form of a perforation is provided near the distal end and near the proximal end of the label 8, respectively.

In the exemplary embodiment depicted in Figure 3, the label 8 has a length suitable for covering not only the housing 2 and the cartridge holder 3 but further the dose setting member 5 and the threaded interface 7 when being heat shrunk onto the injection device 1. Thus, the label 8 forms a first cap portion 11 covering the threaded interface 7 and a second cap portion 12 covering the dose setting member 5. The respective weakened lines 10 are designed to permit the severance and removal of the cap portions 11, 12 from the rest of the label 8. In other words, the label 8 further forms a tamper-evident closure for the injection device 1.

While the embodiment of Figure 3 has been explained with two weakened lines 10 forming to respective cap portions of the label 8, alternative embodiments may comprise only a single weakened line 10 or a may be devoid of a weakened line 10.

Although not depicted in Figure 3, the heat shrinkable label 8 may be used to position, fix and/or maintain component parts in and/or on the disposable injection device 1. Especially, the heat shrinkable label 8 may be used to maintain an electronic component part, e.g. a temperature sensing strip, on the housing 2 or the cartridge holder 3.

### Reference Numerals

- 1: injection device
- 2: housing
- 3: cartridge holder
- 4: cap
- 5: dose setting member
- 6: window
- 7: interface
- 8: heat shrinkable label
- 9: scale
- 10: weakened line
- 11: first cap portion
- 12: second cap portion

## Claims

1. An injection device, comprising
- a housing (2) containing a dose setting mechanism,
- a cartridge holder (3) containing a cartridge with a medicament, and
- a heat shrinkable label (8) which comprises information (9) and/or symbols about the medicament,
**characterized in that** the cartridge holder (3) is inseparably locked to the housing (2),
and **in that** the heat shrinkable label (8) is heat shrunk on the housing (2) and the cartridge holder (3) such that it at least partially covers the housing (2) and the cartridge holder (3).

2. The injection device according to claim 1, **characterized in that** the heat shrinkable label (8) has the form of a jacket at least partially encasing the housing (2) and the cartridge holder (3).

3. The injection device according to claim 1 or 2, **characterized in that** the heat shrinkable label (8) comprises at least a transparent or translucent portion, which is positioned on the housing (2) overlapping a window (6) provided in the housing (2).

4. The injection device according to any one of the preceding claims, **characterized in that** the information (9) and/or symbols are printed on the inner surface of the heat shrinkable label (8).

5. The injection device according to any one of the preceding claims, **characterized in that** the information and/or symbols comprises a scale (9) positioned on the cartridge holder (3) and information about the medicament positioned on the housing (2).

6. The injection device according to any one of the preceding claims, **characterized in that** the outer surface of the heat shrinkable label (8) is partially lacquered.

7. The injection device according to any one of the preceding claims, **characterized in that** the heat shrinkable label (8) consists of PET or OPP.

8. The injection device according to any one of the preceding claims, **characterized in that** the heat shrinkable label (8) consists of and/or is provided with a layer inhibiting the transmission of actinic light.

9. The injection device according to any one of the preceding claims, **characterized in that** the heat shrinkable label (8) comprises an electronic component and/or maintains an electronic component on the housing (2) and/or on the cartridge holder (3).

10. The injection device according to claim 9, **characterized in that** the heat shrinkable label (8) comprises a temperature measuring strip.

11. The injection device according to any one of the preceding claims, **characterized in that** the heat shrinkable label (8) comprises at least one weakened line (10) permitting severance and removal of a portion (11, 12) of the heat shrinkable label (8).

12. The injection device according to claim 11, **characterized in that** the cartridge holder (3) comprises an interface (7) for attaching a needle on the cartridge holder (3), wherein the heat shrinkable label (8) comprises a first cap portion (11) covering the interface (7) for attaching a needle and wherein the at least one weakened line (10) is positioned permitting severance and removal of the first cap portion (11).

13. The injection device according to claim 11 or 12, **characterized in that** the dose setting mechanism comprises a dose setting member (5) protruding from the housing (2), wherein the heat shrinkable label (8) comprises a second cap portion (12) covering the dose setting member (5) and wherein the at least one weakened line (10) is positioned permitting severance and removal of the second cap portion (12).

14. The injection device according to any one of the preceding claims, **characterized in that** the heat shrinkable label (8) is glued or welded onto the housing (2) and/or onto the cartridge holder (3).

15. The injection device according to any one of the preceding claims, **characterized in that** the medicament is an insulin or a growth hormone.

## Patentansprüche

1. Injektionsvorrichtung, aufweisend:
- ein Gehäuse (2), das einen Dosiseinstellmechanismus enthält,
- einen Kartuschenhalter (3), der eine Kartusche mit einem Medikament enthält, und
- ein heißschrumpffähiges Etikett (8), das Informationen (9) und/oder Symbole über das Medikament aufweist,
**dadurch gekennzeichnet, dass** der Kartuschenhalter (3) untrennbar mit dem Gehäuse (2) verriegelt ist
und dass das heißschrumpffähige Etikett (8) so auf das Gehäuse (2) und den Kartuschenhalter (3) heißaufgeschrumpft ist, dass es das Gehäuse (2) und den Kartuschenhalter (3) zumindest teilweise abdeckt.

2. Injektionsvorrichtung nach Anspruch 1, **dadurch gekennzeichnet, dass** das heißschrumpffähige Etikett (8) die Form eines Mantels hat, der das Gehäuse (2) und den Kartuschenhalter (3) zumindest teilweise einschließt.

3. Injektionsvorrichtung nach Anspruch 1 oder 2, **dadurch gekennzeichnet, dass** das heißschrumpffähige Etikett (8) zumindest einen transparenten oder durchscheinenden Abschnitt aufweist, der auf dem Gehäuse (2) positioniert ist und ein in dem Gehäuse (2) vorgesehenes Fenster (6) überlappt.

4. Injektionsvorrichtung nach einem der vorhergehenden Ansprüche, **dadurch gekennzeichnet, dass** die Informationen (9) und/oder Symbole auf der Innenfläche des heißschrumpffähigen Etiketts (8) aufgedruckt sind.

5. Injektionsvorrichtung nach einem der vorhergehenden Ansprüche, **dadurch gekennzeichnet, dass** die Informationen und/oder Symbole eine auf dem Kartuschenhalter (3) positionierte Skala (9) und auf dem Gehäuse (2) positionierte Informationen über das Medikament aufweisen.

6. Injektionsvorrichtung nach einem der vorhergehenden Ansprüche, **dadurch gekennzeichnet, dass** die Außenfläche des heißschrumpffähigen Etiketts (8) teilweise lackiert ist.

7. Injektionsvorrichtung nach einem der vorhergehenden Ansprüche, **dadurch gekennzeichnet, dass** das heißschrumpffähige Etikett (8) aus PET oder OPP besteht.

8. Injektionsvorrichtung nach einem der vorhergehenden Ansprüche, **dadurch gekennzeichnet, dass** das heißschrumpffähige Etikett (8) aus einer Schicht besteht, die das Durchlassen von aktinischem Licht unterbindet, und/oder mit einer solchen versehen ist.

9. Injektionsvorrichtung nach einem der vorhergehenden Ansprüche, **dadurch gekennzeichnet, dass** das heißschrumpffähige Etikett (8) eine elektronische Komponente aufweist und/oder eine elektronische Komponente an dem Gehäuse (2) und/oder an dem Kartuschenhalter (3) hält.

10. Injektionsvorrichtung nach Anspruch 9, **dadurch gekennzeichnet, dass** das heißschrumpffähige Etikett (8) einen Temperaturmessstreifen aufweist.

11. Injektionsvorrichtung nach einem der vorhergehenden Ansprüche, **dadurch gekennzeichnet, dass** das heißschrumpffähige Etikett (8) zumindest eine Schwächungslinie (10) aufweist, die das Abtrennen und das Entfernen eines Abschnitts (11, 12) des heißschrumpffähigen Etiketts (8) gestattet.

12. Injektionsvorrichtung nach Anspruch 11, **dadurch gekennzeichnet, dass** der Kartuschenhalter (3) eine Schnittstelle (7) zum Anbringen einer Nadel an dem Kartuschenhalter (3) aufweist, wobei das heißschrumpffähige Etikett (8) einen ersten Kappenabschnitt (11) aufweist, der die Schnittstelle (7) zum Anbringen einer Nadel abdeckt, und wobei die zumindest eine Schwächungslinie (10) positioniert ist und das Abtrennen und das Entfernen des ersten Kappenabschnitts (11) gestattet.

13. Injektionsvorrichtung nach Anspruch 11 oder 12, **dadurch gekennzeichnet, dass** der Dosiseinstellmechanismus ein Dosiseinstellglied (5) aufweist, das von dem Gehäuse (2) vorragt, wobei das heißschrumpffähige Etikett (8) einen zweiten Kappenabschnitt (12) aufweist, der das Dosiseinstellglied (5) abdeckt, und wobei die zumindest eine Schwächungslinie (10) positioniert ist und das Abtrennen und das Entfernen des zweiten Kappenabschnitts (12) gestattet.

14. Injektionsvorrichtung nach einem der vorhergehenden Ansprüche, **dadurch gekennzeichnet, dass** das heißschrumpffähige Etikett (8) auf das Gehäuse (2) und/oder auf den Kartuschenhalter (3) geklebt oder geschweißt ist.

15. Injektionsvorrichtung nach einem der vorhergehenden Ansprüche, **dadurch gekennzeichnet, dass** das Medikament ein Insulin oder ein Wachstumshormon ist.

## Revendications

1. Dispositif d'injection, comprenant
- un boîtier (2) contenant un mécanisme de réglage de dose,
- un support de cartouche (3) contenant une cartouche avec un médicament, et
- une étiquette thermorétractable (8) qui comprend des informations (9) et/ou des symboles concernant le médicament,
**caractérisé en ce que** le support de cartouche (3) est verrouillé de manière indissociable au boîtier (2),
et **en ce que** l'étiquette thermorétractable (8) est thermorétractée sur le boîtier (2) et le support de cartouche (3) de sorte qu'elle recouvre au moins partiellement le boîtier (2) et le support de cartouche (3) .

2. Dispositif d'injection selon la revendication 1, **caractérisé en ce que** l'étiquette thermorétractable (8) se présente sous la forme d'une gaine enveloppant au moins partiellement le boîtier (2) et le support de cartouche (3).

3. Dispositif d'injection selon la revendication 1 ou 2, **caractérisé en ce que** l'étiquette thermorétractable (8) comprend au moins une partie transparente ou translucide, qui est positionnée sur le boîtier (2), chevauchant une fenêtre (6) située dans le boîtier (2).

4. Dispositif d'injection selon l'une quelconque des revendications précédentes, **caractérisé en ce que** les informations (9) et/ou symboles sont imprimé(e)s sur la surface intérieure de l'étiquette thermorétractable (8).

5. Dispositif d'injection selon l'une quelconque des revendications précédentes, **caractérisé en ce que** les informations et/ou symboles comprennent une échelle (9) positionnée sur le support de cartouche (3) et des informations concernant le médicament positionnées sur le boîtier (2).

6. Dispositif d'injection selon l'une quelconque des revendications précédentes, **caractérisé en ce que** la surface extérieure de l'étiquette thermorétractable (8) est partiellement laquée.

7. Dispositif d'injection selon l'une quelconque des revendications précédentes, **caractérisé en ce que** l'étiquette thermorétractable (8) est constituée de PET ou OPP.

8. Dispositif d'injection selon l'une quelconque des revendications précédentes, **caractérisé en ce que** l'étiquette thermorétractable (8) est constituée et/ou est pourvue d'une couche inhibant la transmission de lumière actinique.

9. Dispositif d'injection selon l'une quelconque des revendications précédentes, **caractérisé en ce que** l'étiquette thermorétractable (8) comprend un composant électronique et/ou maintient un composant électronique sur le boîtier (2) et/ou sur le support de cartouche (3).

10. Dispositif d'injection selon la revendication 9, **caractérisé en ce que** l'étiquette thermorétractable (8) comprend une bande de mesure de la température.

11. Dispositif d'injection selon l'une quelconque des revendications précédentes, **caractérisé en ce que** l'étiquette thermorétractable (8) comprend au moins une ligne affaiblie (10) permettant de sectionner et de retirer une partie (11, 12) de l'étiquette thermorétractable (8).

12. Dispositif d'injection selon la revendication 11, **caractérisé en ce que** le support de cartouche (3) comprend une interface (7) pour fixer une aiguille sur le support de cartouche (3), l'étiquette thermorétractable (8) comprenant une première partie de capuchon (11) recouvrant l'interface (7) pour fixer une aiguille, et l'au moins une ligne affaiblie (10) étant positionnée de sorte à permettre de sectionner et de retirer la première partie de capuchon (11).

13. Dispositif d'injection selon la revendication 11 ou 12, **caractérisé en ce que** le mécanisme de réglage de dose comprend un élément de réglage de dose (5) faisant saillie à partir du boîtier (2), l'étiquette thermorétractable (8) comprenant une deuxième partie de capuchon (12) recouvrant l'élément de réglage de dose (5), et l'au moins une ligne affaiblie (10) étant positionnée de sorte à permettre de sectionner et de retirer la deuxième partie de capuchon (12).

14. Dispositif d'injection selon l'une quelconque des revendications précédentes, **caractérisé en ce que** l'étiquette thermorétractable (8) est collée ou soudée sur le boîtier (2) et/ou sur le support de cartouche (3).

15. Dispositif d'injection selon l'une quelconque des revendications précédentes, **caractérisé en ce que** le médicament est une insuline ou une hormone de croissance.
